# FASCICULE DE BREVET EUROPEEN

(11) **EP 0 426 557 B1**
(45) Date de publication et mention de la délivrance du brevet: **22.02.1995**
(21) Numéro de dépôt: 90403061.6
(22) Date de dépôt: 30.10.1990
(51) Int. Cl.: C07D 409/04, A61K 31/44, C12P 17/16

(54) **Procédé de préparation de (pyridyl-3)-2 tétrahydrothiopyrannecarbothioamide-2-oxydes-1-(1R,2R), les (pyridyl-3)-2 tétrahydrothiopyrannecarbothioamide-2-oxydes-1-(1R,2R) ainsi obtenus et les compositions pharmaceutiques qui les contiennent**
Verfahren zur Herstellung von 2-(3-Pyridyl)-Tetrahydrothiopyran-2-carbonsäurethioamid-1-(1R,2R)oxyden, die so erhaltenen 2-(3-Pyridyl)-Tetrahydrothiopyran-2-carbothioamid-1-(1R,2R)oxyde und diese enthaltende pharmazeutische Zusammenstellungen
Process for the preparation of 2-(3-pyridyl)-tetrahydrothiopyran-2-carbothiamide-1-(1R,2R)oxydes, the 2-(3-pyridyl)-tetrahydrothiopyran-2-carbothiamide-1-(1R,2R)oxydes and so obtained and pharmaceutical compositions containing them

(30) Priorité: 31.10.1989 FR 8914273
(43) Date de publication de la demande: 08.05.1991
(73) Titulaire: RHONE-POULENC SANTE, 92160 Antony (FR)
(72) Inventeur: Aloup, Jean-Claude, F-94290 Villeneuve Le Roi (FR); James, Claude, F-75020 Paris (FR); Margraff, Rodolphe, F-91170 Viry Chatillon (FR)
(74) Mandataire: Pilard, Jacques

(56) Documents cités:
- EP-A- 0 097 584
- EP-A- 0 135 638

## Description

La présente invention concerne un procédé de préparation de (pyridyl-3)-2 tétrahydrothiopyrannecarbothioamide-2-oxydes-1-(1R,2R) qui présentent des propriétés anti-hypertensives particulièrement intéressantes.

Dans le brevet européen EP 0097584 ont été décrits des dérivés du thioformamide de formule générale : 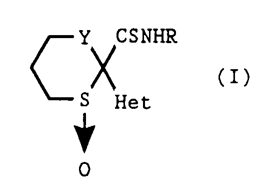
dans laquelle R représente un atome d'hydrogène ou un radical alcoyle contenant 1 à 4 atomes de carbone, Het représente un radical hétérocyclique à caractère aromatique et Y représente une liaison de valence ou un radical méthylène.

La présence de deux centres d'asymétrie conduit à 4 stéréoisomères pouvant être éventuellement séparés en 2 couples racémiques qui ont été désignés par "Forme A" (ou produit le plus polaire) et "Forme B" (ou produit le moins polaire) [la polarité étant déterminée par chromatographie sur couche mince (CCM)]. Ces deux formes peuvent être dédoublées.

Parmi les produits de formule générale (I), la forme A du N-méthyl(pyridyl-3)-2 tétrahydrothiopyrannecarbothioamide-2-oxyde-1 est constituée du mélange des isomères trans qui peuvent être représentés de la manière suivante : 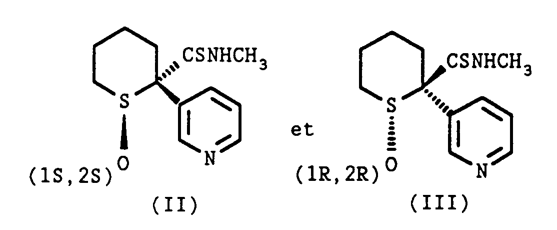
Les études effectuées sur les isomères (II) et (III) ont permis de montrer que la forme active est l'isomère (III) dont la configuration absolue est 1R,2R.

La présente invention concerne un procédé de préparation des dérivés 1R,2R du thioformamide de formule générale : 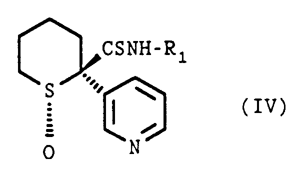
dans laquelle R₁ représente un radical alcoyle droit ou ramifié contenant 1 à 4 atomes de carbone.

Selon l'invention, les produits de formule générale (IV) peuvent être obtenus par action d'un isothiocyanate d'alcoyle de formule générale :

S=C=N-R₁ (V)

dans laquelle R₁ est défini comme précédemment, sur un sulfoxyde de formule : 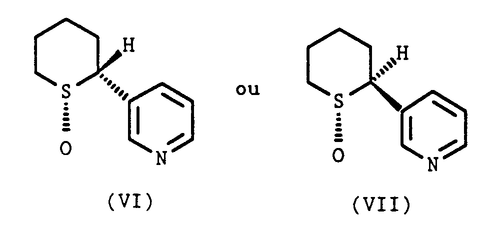
préalablement anionisé.

Généralement, la réaction est effectuée en ajoutant une solution d'un sulfoxyde de formule (VI) ou (VII) ou d'un mélange des sulfoxydes de formule (VI) et (VII) dans un solvant organique inerte tel qu'un éther comme le tétrahydrofuranne à de l'amidure de sodium (éventuellement préparé in situ) dans l'ammoniac liquide en opérant à la température d'ébullition du mélange réactionnel, c'est-à-dire à -30°C, puis en ajoutant une solution d'un isothiocyanate de formule générale (V) dans un solvant organique inerte tel qu'un éther comme le tétrahydrofuranne à la même température.

Les sulfoxydes de formule (VI) ou (VII) ou leurs mélanges peuvent être obtenus par oxydation stéréosélective d'un produit de formule : 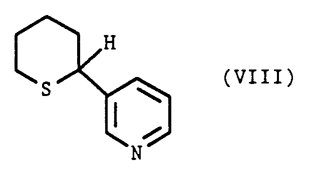
qui se présente généralement sous forme d'un mélange racémique R,S.

L'oxydation par les méthodes classiques non stéréosélectives d'un produit de formule (VIII) conduit au mélange des sulfoxydes de formule (VI) et (VII) et des sulfoxydes de formule : 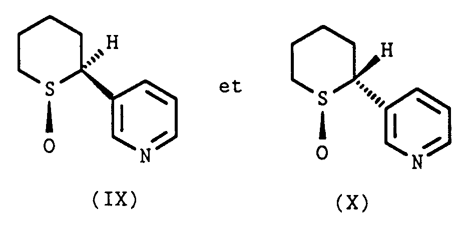
Seuls les sulfoxydes de formule (VI) et (VII) peuvent être utilisés pour obtenir les énantiomères actifs qui ont la configuration 1R,2R.

L'oxydation sélective d'un produit de formule (VIII) peut être réalisée soit par voie chimique soit par voie biochimique.

Généralement l'oxydation sélective par voie chimique est réalisée en présence d'un inducteur d'asymétrie tel que le (+)-tartrate de diéthyle et d'un dérivé du titane (IV) tel qu'un alcoolate de titane comme l'isopropylate de titane (IV) au moyen d'un hydroperoxyde tel que l'hydroperoxyde de cumyle ou de tert.butyle. Généralement on opère dans un solvant organique tel qu'un hydrocarbure aliphatique halogéné comme le chlorure de méthylène ou le dichloro-1,2 éthane. L'oxydation est, de préférence, effectuée à une température voisine de -20°C.

Les produits de formule (VI) et (VII) ainsi obtenus peuvent être séparés et purifiés par chromatographie sur un support approprié.

Généralement l'oxydation sélective par voie biochimique est réalisée au moyen d'une culture d'un champignon filamenteux ou d'une bactérie filamenteuse ou au moyen d'une enzyme isolée en présence d'un agent d'oxydation [H.L. Holland, Chemical Reviews, 88, 473-485 (1988)]. De préférence on utilise Aspergillus foetidus NRRL 337. L'oxydation est effectuée en ajoutant une solution stérile du produit de formule (VIII) soit à une culture du microorganisme dans un milieu approprié ayant atteint un degré suffisant de développement puis en poursuivant l'incubation jusqu'à l'obtention d'un taux convenable de transformation du produit de formule (VIII), soit à une solution de l'enzyme contenant un agent d'oxydation tel que l'eau oxygénée ou l'hydroperoxyde de tert.butyle.

Les produits de formule (VI) et (VII) sont séparés du milieu de culture dans les conditions habituelles et ils sont purifiés par chromatographie sur des supports appropriés.

La présente invention concerne également les sulfoxydes de formule (VI) et (VII).

Le produit de formule (VIII) peut être obtenu selon l'une des méthodes suivantes, c'est-à-dire :
- soit par décarboxylation de l'acide de formule : 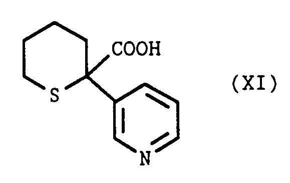 par chauffage à une température comprise entre 130 et 160°C, l'acide de formule (XI) étant obtenu dans les conditions décrites dans le brevet européen EP 0 073 704.
- soit par cyclisation d'un produit de formule générale : 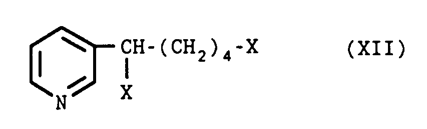 éventuellement sous forme de sel, dans laquelle X représente un atome d'halogène (chlore, brome) ou un reste d'ester réactif (méthylsulfonyloxy) au moyen de sulfure de sodium en opérant dans un milieu hydro-organique biphasique en présence d'un catalyseur de transfert de phase tel qu'un halogénure de tétraalkylammonium comme le bromure de tétrabutylammonium.

Le produit de formule générale (XII) peut être obtenu par action d'un agent d'halogénation (chlorure de thionyle), ou d'un agent d'estérification (chlorure de méthanesulfonyle) sur le diol de formule: 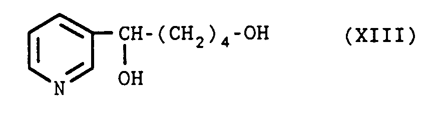
Généralement, lorsqu'on utilise un agent d'halogénation, on opère dans un solvant organique choisi parmi les hydrocarbures aliphatiques halogénés tel que le chlorure de méthylène ou le chloroforme à une température comprise entre 0 et 50°C et, lorsqu'on utilise un agent d'estérification, on opère en présence d'un agent basique (pyridine, triéthylamine) à une température voisine de 0°C.

Le produit de formule (XIII) peut être obtenu par réduction du céto-alcool de formule : 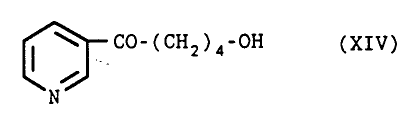
Généralement la réduction est effectuée au moyen d'un borohydrure alcalin, tel que le borohydrure de sodium, en opérant en milieu hydro-alcoolique à une température voisine de 0°C.

Le céto-alcool de formule (XIV) peut être obtenu par action de la lithio-3 pyridine sur la δ-valérolactone, la lithio-3 pyridine pouvant être obtenue par action d'un agent de métallation tel que le butyllithium sur une halogéno-3 pyridine telle que la bromo-3 pyridine.

Généralement on opère dans un solvant organique inerte tel qu'un éther (éther éthylique, tétrahydrofuranne) éventuellement en présence d'un hydrocarbure aliphatique (hexane) à une température inférieure à -50°C.
- soit par réduction des formes (1RS,2RS) et/ou (1RS,2SR) dérivées du sulfoxyde de formule : 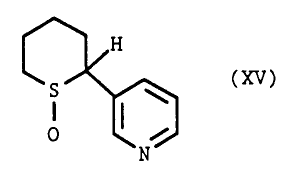 au moyen d'un agent réducteur des sulfoxydes tel qu'un hydrogénosulfite alcalin, comme l'hydrogénosulfite de sodium en solution aqueuse.

Les formes (1RS,2RS) et (1RS,2SR) dérivées du sulfoxyde de formule (XV) peuvent être obtenues par cyclisation d'un produit de formule : 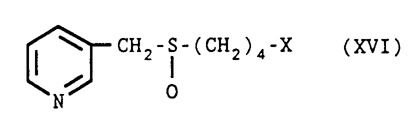
dans laquelle X est défini comme précédemment, au moyen d'une base telle qu'un alcoolate alcalin (tert.butylate de potassium) en opérant dans un solvant organique inerte tel qu'un éther comme le tétrahydrofuranne.

Les formes (1RS,2RS) et (1RS,2SR) dérivées du produit de formule (XV) peuvent être séparées par chromatographie sur des supports appropriés.

Le produit de formule (XVI) peut être préparé dans les conditions décrites dans les brevet européen EP 0 097 584.

Les exemples suivants, donnés à titre non limitatif, montrent comment l'invention peut être mise en pratique.

### EXEMPLE 1

A une solution de 0,05 g de nitrate ferrique dans 4 cm3 d'ammoniac liquide maintenue à -40°C sous atmosphère d'azote est ajouté 0,24 g de sodium. La solution est agitée pendant 15 minutes à la même température puis on ajoute successivement une solution de 1 g de (-)(pyridyl-3)-2 tétrahydrothiopyranne-oxyde-1-(1R,2R) ([α]²⁰_{D} = -219° ± 2 ; c = 1, chloroforme) dans 10 cm3 de tétrahydrofuranne anhydre en une minute puis une solution de 0,5 g d'isothiocyanate de méthyle dans 2 cm3 de tétrahydrofuranne anhydre en quelques secondes. On agite pendant 10 minutes à une température comprise entre -40°C et -35°C, ajoute 0,6 g de chlorure d'ammonium, laisse remonter progressivement la température au voisinage de 20°C puis concentre à sec sous pression réduite (25 mm de mercure ; 3,4 kPa) à 30°C.

Le mélange obtenu, additionné de 10 cm3 d'une solution aqueuse saturée de chlorure de sodium, est extrait 3 fois par 45 cm3 au total de chlorure de méthylène et les extraits organiques réunis sont séchés sur du sulfate de magnésium anhydre, filtrés et concentrés à sec sous pression réduite (25 mm de mercure ; 3,4 kPa) à 30°C.

Le produit obtenu (1,1 g) est dissous dans 130 cm3 d'acétate d'éthyle bouillant. La solution est filtrée à chaud, refroidie puis conservée pendant 2 heures à une température voisine de 5°C. Les cristaux apparus sont séparés par filtration et séchés sous pression réduite (2 mm de mercure ; 0,27 kPa) à 40°C.

On obtient ainsi 0,45 g de (-) N-méthyl (pyridyl-3)-2 tétrahydrothiopyrannecarbothioamide-2-oxyde-1-(1R,2R) fondant à 244°C dont le pouvoir rotatoire est :
[α]²⁰_{D} = -207,7° ± 1,9 ; c = 1, chloroforme.

### EXEMPLE 2 - Préparation du (pyridyl-3)-2 tétrahydrothiopyranne-oxyde-1 - Oxydation chimique

A une solution de 18 g de (+) tartrate de diéthyle dans 400 cm3 de chlorure de méthylène anhydre et exempt d'éthanol maintenue sous atmosphère d'azote, on ajoute en agitant à une température voisine de 20°C, 12,4 g d'isopropylate de titane (IV) puis 0,8 g d'eau distillée. On agite pendant 25 minutes, refroidit à -20°C, ajoute 7,8g de (pyridyl-3)-2 tétrahydrothiopyranne-(R,S) puis goutte à goutte en 15 minutes 8,5 g d'hydroperoxyde de cumyle à 82 %. On agite pendant 20 heures à -20°C puis, après addition de 20 cm3 d'eau distillée, pendant 1 heure en laissant remonter progressivement la température à 20°C. Le mélange est filtré et l'insoluble est lavé 3 fois par 450 cm3 au total de chlorure de méthylène. Le filtrat et les lavages réunis sont lavés par 100 cm3 de soude N, par 200 cm3 d'une solution aqueuse saturée de chlorure de sodium, séchés sur du sulfate de sodium anhydre, filtrés et concentrés à sec sous pression réduite (30 mm de mercure ; 4,1 kPa) à 40°C.

Le produit obtenu (18 g) est chromatographié sur 180 g de gel de silice neutre (0,063-0,200 mm) contenus dans une colonne de 4 cm de diamètre. On élue par un mélange d'acétate d'éthyle et de méthanol (97-3 en volumes) en recueillant des fractions de 120 cm3. Les fractions 17 à 29 sont réunies et concentrées à sec sous pression réduite (30 mm de mercure ; 4,1 kPa) à 30°C.

Le produit obtenu (1,5 g) est dissous dans 4,5 cm3 d'acétate d'éthyle bouillant et la solution, après refroidissement, est conservée pendant 2 heures à une température voisine de 5°C. Les cristaux apparus sont séparés par filtration, lavés par 1 cm3 d'acétate d'éthyle et séchés sous pression réduite (2 mm de mercure; 2,6 kPa) à 45°C. On obtient 1,3 g de produit dont on dissout 1,1 g dans 5,5 cm3 d'acétate d'éthyle bouillant. La solution, après refroidissement, est conservée pendant 2 heures à une température voisine de 5°C. Les cristaux apparus sont séparés par filtration et séchés sous pression réduite (2 mm de mercure ; 0,27 kPa) à 45°C.

On obtient ainsi 1,1 g de (pyridyl-3)-2 tétrahydrothiopyranne-oxyde-1-(1R,2R) fondant à 129°C dont le pouvoir rotatoire est:
[α]²⁰_{D} = -219° ± 2 ; c = 1, chloroforme.

Les fractions 46 à 60 sont réunies et concentrées à sec sous pression réduite (30 mm de mercure ; 4,1 kPa) à 30°C.

On obtient ainsi 3,0 g d'un mélange des énantiomères (1R,2S) et (1S,2R) du (pyridyl-3)-2 tétrahydrothiopyranne-oxyde-1 fondant à 109°C dont le pouvoir rotatoire est :
[α]²⁰_{D} = -144° ± 1,6 ; c = 1, chloroforme.

Le (pyridyl-3)-2 tétrahydrothiopyranne-(R,S) peut être préparé selon l'une des méthodes suivantes :
1) 32 g d'acide (pyridyl-3)-2 tétrahydrothiopyrannecarboxylique-2 sont chauffés pendant 45 minutes à une température voisine de 140°C. Après refroidissement, le produit est chromatographié sur 200 g de gel de silice neutre (0,060-0,200 mm) contenus dans une colonne de 4 cm de diamètre. On élue la colonne par du chlorure de méthylène en recueillant des fractions de 250 cm3. Les fractions 4 à 14 sont réunies et concentrées à sec sous pression réduite (25 mm de mercure ; 3,4 kPa) à 40°C. On obtient ainsi 12,5 g de (pyridyl-3)-2 tétrahydrothiopyranne-(R,S) fondant à 49°C.
   L'acide (pyridyl-3)-2 tétrahydrothiopyrannecarboxylique-2 peut être préparé selon la méthode décrite dans le brevet européen EP 0 073 704.
2) A une solution de 1,25 g de soude dans 1,25 g d'eau, on ajoute successivement, en agitant à 20°C, 20 cm3 de toluène, 0,06 g de bromure de tétrabutylammonium, 3,6 g de sulfure de sodium nonahydrate puis 2,5g de chlorhydrate de dichloro-1,5 (pyridyl-3)-5 pentane. Le mélange est agité pendant 1 heure 30 minutes à 70°C puis, après refroidissement, on ajoute 10 cm3 d'eau distillée. Après décantation, la phase aqueuse est extraite 4 fois par 80 cm3 au total d'éther. Les extraits organiques sont réunis, lavés 2 fois par 50 cm3 au total d'eau distillée, séchés sur du sulfate de sodium anhydre et concentrés à sec sous pression réduite (25 mm de mercure ; 3,4 kPa) à 30°C.
   Le produit ainsi obtenu (1,5 g) est chromatographié sur 7,5 g de gel de silice neutre (0,063-0,200 mm) contenus dans une colonne de 1,5 cm de diamètre. La colonne est éluée par de l'acétate d'éthyle en recueillant des fractions de 100 cm3. La fraction 1 est concentrée à sec sous pression réduite (25 mm de mercure ; 3,4 kPa) à 50°C.
   On obtient ainsi 1,1 g de (pyridyl-3)-2 tétrahydrothiopyranne-(R,S) fondant à 49°C.
   Le chlorhydrate de dichloro-1,5 (pyridyl-3)-5 pentane peut être préparé de la façon suivante :
   A une solution de 10,9 g de (pyridyl-3)-5 pentanediol-1,5 dans 90 cm3 de chloroforme on ajoute goutte à goutte en 10 minutes 21,4 g de chlorure de thionyle à une température comprise entre 28°C et 48°C. Le mélange est ensuite maintenu à l'ébullition pendant 2 heures jusqu'à la fin du dégagement gazeux, refroidi à 20°C, conservé à cette température pendant 16 heures. Après concentration à sec sous pression réduite (25 mm de mercure ; 3,4 kPa) à 35°C, on obtient un résidu pesant 14,5 g.
   On dissout les 14,5 g du produit obtenu dans les conditions décrites ci-dessus dans un mélange bouillant de 50 cm3 d'oxyde d'isopropyle et 75 cm3 d'isopropanol. La solution, additionnée de noir décolorant, est filtrée à chaud et le filtre est lavé 3 fois par 300 cm3 au total d'isopropanol bouillant. Après addition de 700 cm3 d'oxyde d'isopropyle, le mélange est refroidi et est conservé pendant 2 heures à une température voisine de 5°C. Les cristaux apparus sont séparés par filtration, lavés 3 fois par 300 cm3 au total d'oxyde d'isopropyle et séchés sous pression réduite (25 mm de mercure; 3,4 kPa) à 20°C.
   On obtient ainsi 13,9 g de chlorhydrate de dichloro-1,5 (pyridyl-3)-5 pentane fondant à 123°C.
   Le (pyridyl-3)-5 pentanediol-1,5 peut être préparé de la façon suivante :
   A une solution de 25,7 g d'oxo-5 (pyridyl-3)-5 pentanol-1 dans 270 cm3 de méthanol maintenue à une température voisine de 0°C, on ajoute goutte à goutte en 20 minutes une solution de 26,5 g de borohydrure de sodium dans 270 cm3 d'un mélange d'eau et de méthanol (50-50 en volumes). Le mélange est ensuite agité pendant 22 heures à une température voisine de 20°C puis est concentré à sec sous pression réduite (25 mm de mercure ; 3,4 kPa) à 40°C.
   Le produit obtenu est dissous dans 200 cm3 d'eau distillée et la solution est saturée par du chlorure de sodium. L'huile qui décante en couche supérieure est séparée et est dissoute dans 50 cm3 de méthanol, séchée sur du sulfate de sodium anhydre, filtrée et concentrée à sec sous pression réduite (25 mm de mercure ; 3,4 kPa) à 50°C. On obtient ainsi un premier lot de 18,2 g. La phase aqueuse inférieure est extraite 3 fois par 750 cm3 au total de chloroforme et les extraits organiques sont réunis, séchés sur du sulfate de sodium anhydre, filtrés et concentrés à sec sous pression réduite (25 mm de mercure ; 3,4 kPa) à 50°C. On obtient ainsi un deuxième lot de 5,6 g.
   Le mélange de ces deux lots est chromatographié sur 360 g de gel de silice (0,063-0,200 mm) contenus dans une colonne de 4,7 cm de diamètre. On élue par de l'acétate d'éthyle en recueillant des fractions de 80 cm3. Les fractions 44 à 72 sont réunies et concentrées à sec sous pression réduite (25 mm de mercure ; 3,4 kPa) à 50°C. On obtient ainsi 16,8 g de (pyridyl-3)-5 pentanediol-1,5 sous la forme d'une huile jaune. [Rf = 0,4 ; chromatographie sur couche mince de gel de silice ; solvant : acétate d'éthyle-méthanol (80-20 en volumes)].
   L'oxo-5 (pyridyl-3)-5 pentanol-1 peut être préparé de la façon suivante :
   A 70 cm3 d'une solution 1,6M de n-butyllithium dans l'hexane maintenue sous atmosphère d'azote à une température voisine de -70°C, on ajoute goutte à goutte en 20 minutes une solution de 17,7 g de bromo-3 pyridine dans 100 cm3 d'éther anhydre. Après 30 minutes d'agitation à la même température, on ajoute goutte à goutte en 20 minutes une solution de 11,2 g de δ-valérolactone dans 200 cm3 d'éther anhydre. Le mélange est ensuite agité pendant 1 heure à une température voisine de -70°C puis pendant 2 heures 15 minutes en laissant remonter progressivement la température à 16°C. On ajoute goutte à goutte 150 cm3 d'eau distillée à une température voisine de 20°C. Après décantation, la phase aqueuse est extraite 3 fois par 750 cm3 au total d'acétate d'éthyle. Les phases organiques réunies sont lavées 2 fois par 500 cm3 au total d'eau distillée, séchées sur du sulfate de sodium anhydre, filtrées et concentrées à sec sous pression réduite (25 mm de mercure ; 3,4 kPa) à 45°C.
   Le produit ainsi obtenu (18,1 g) est chromatographié sur 250 g de gel de silice neutre (0,063-0,200 mm) contenus dans une colonne de 4,7 cm de diamètre. On élue la colonne par un mélange cyclohexane-acétate d'éthyle (50-50 en volumes) en recueillant des fractions de 90 cm3. Les fractions 44 à 58 sont réunies et concentrées à sec sous pression réduite (22 mm de mercure; 3 kPa) à 40°C.
   On obtient ainsi 13,3 g d'oxo-5 (pyridyl-3)-5 pentanol-1 sous la forme d'une huile jaune. (Rf = 0,18 ; chromatographie sur couche mince de gel de silice ; solvant : acétate d'éthyle).
3) 3,9 g de (pyridyl-3)-2 tétrahydrothiopyranne-oxyde-1-(1RS,2RS) sont dissous dans 25 cm3 d'une solution aqueuse à 37,5 % d'hydrogénosulfite de sodium. Après chauffage à l'ébullition pendant 22 heures et refroidissement, la solution est extraite 4 fois par 100 cm3 au total de chlorure de méthylène et les extraits organiques réunis sont séchés sur du sulfate de sodium anhydre, filtrés et concentrés à sec sous pression réduite (25 mm de mercure ; 3,4 kPa) à 35°C.

Le produit obtenu (2,9 g) est chromatographié sur 325 g de gel de silice neutre (0,040-0,063 mm) contenus dans une colonne de 5,5 cm de diamètre. On élue sous pression réduite (200 mm de mercure ; 25 kPa) par un mélange de cyclohexane et d'acétate d'éthyle (65-35 en volumes) en recueillant des fractions de 100 cm3. Les fractions 11 à 27 sont réunies et concentrées à sec sous pression réduite (25 mm de mercure ; 3,4 kPa) à 35°C.

On obtient ainsi 2,7 g de (pyridyl-3)-2 tétrahydrothiopyranne-(R,S) fondant à 49°C.

Le (pyridyl-3)-2 tétrahydrothiopyranne-oxyde-1-(1RS,2RS) peut être préparé de la manière suivante :
A une solution de 70,2 g de t-butylate de potassium dans 380 cm3 de tétrahydrofuranne anhydre maintenue sous atmosphère d'azote à une température voisine de 0°C, on ajoute goutte à goutte en 2 heures une solution de [(chloro-4 butyl)-sulfinylméthyl]-3 pyridine dans 180 cm3 de tétrahydrofuranne anhydre. Le mélange est ensuite agité pendant 1 heure à la même température puis pendant 16 heures à une température voisine de 20°C, additionné de 20 cm3 d'acide acétique puis filtré. L'insoluble est lavé 4 fois par 580 cm3 au total de chlorure de méthylène et les filtrats réunis sont concentrés à sec sous pression réduite (20 mm de mercure ; 2,7 kPa) à 40°C.

Le produit obtenu (61 g), additionné de 82 g préparés dans les mêmes conditions, est chromatographié sur 600 g de gel de silice neutre (0,063-0,200 mm) contenus dans une colonne de 6 cm de diamètre. On élue par 14,4 litres d'un mélange d'acétate d'éthyle et de méthanol (90-10 en volumes) puis par 3,9 litres d'un mélange d'acétate d'éthyle et de méthanol (80-20 en volumes) en recueillant des fractions de 300 cm3. Les fractions 21 à 35 sont réunies et concentrées à sec sous pression réduite (20 mm de mercure ; 2,7 kPa) à 45°C.

Le produit obtenu (25 g) est dissous dans 125 cm3 d'acétate d'éthyle bouillant et la solution additionnée de noir décolorant est filtrée à chaud. Après refroidissement, la solution est conservée pendant 15 heures à une température voisine de 5°C. Les cristaux apparus sont séparés par filtration, lavés 3 fois par 45 cm3 au total d'acétate d'éthyle et séchés sous pression réduite (0,2 mm de mercure ; 0,027 kPa) à 50°C.

On obtient ainsi 21,2 g de (pyridyl-3)-2 tétrahydrothiopyranne-oxyde-1-(1RS,2RS) fondant à 130°C.

Les fractions 49 à 52 sont réunies et concentrées à sec sous pression réduite (20 mm de mercure ; 2,7 kPa) à 45°C. On obtient ainsi un premier lot de 10,4 g.

Les fractions 53 à 61 sont réunies et concentrées à sec sous pression réduite (20 mm de mercure ; 2,7 kPa) à 45°C. On obtient ainsi un second lot de 9,9 g.

Ces premier et second lots sont recristallisés comme décrit précédemment respectivement dans 90 cm3 et 100 cm3 d'acétate d'éthyle bouillant pour donner deux nouveaux lots de 7,3 g et 5,6 g.

Ces derniers lots sont réunis, dissous dans 155 cm3 d'acétate d'éthyle bouillant et la solution, additionnée de noir décolorant, est filtrée à chaud, refroidie et conservée pendant 16 heures à une température voisine de 5°C. Les cristaux apparus sont séparés par filtration, lavés 3 fois par 30 cm3 au total d'acétate d'éthyle et séchés sous pression réduite (0,2 mm de mercure ; 0,03 kPa) à 50°C.

On obtient ainsi 10,4 g de (pyridyl-3)-2 tétrahydrothiopyranne-oxyde-1-(1RS,2SR) fondant à 120°C.

### EXEMPLE 3 - Préparation du (pyridyl-3)-2 tétrahydrothiopyranne-oxyde-1 - Oxydation biochimique

On prépare un milieu de culture ayant la composition suivante :

| | |
|---|---|
| - glucose | 30 g |
| - phosphate dipotassique | 4 g |
| - nitrate de sodium | 2 g |
| - chlorure de potassium | 0,5 g |
| - sulfate de magnésium | 0,5 g |
| - sulfate de fer | 0,01 g |
| - eau déminéralisée q.s.p. | 1000 cm3 |

On ajuste le pH à 4,3 par addition d'acide chlorhydrique et stérilise à l'autoclave pendant 30 minutes à 121°C le glucose étant stérilisé séparément.

On ensemence 50 cm3 de milieu de culture stérile contenus dans un erlenmeyer de 250 cm3 avec 2 cm3 d'une suspension de spores d'Aspergillus foetidus NRRL 337 provenant d'une culture gélosée inclinée. On incube pendant 3 jours à 28°C sur une table agitée à 200 tours/minute. On obtient ainsi une culture inoculum qui est utilisée pour ensemencer 10 erlenmeyers identiques contenant chacun 50 cm3 du milieu de culture décrit ci-dessus. Chaque erlenmeyer est ensemencé avec 2 cm3 de la culture inoculum. On incube pendant 4 jours à 28°C sur une table agitée à 200 tours/minute.

Dans chacun des 10 erlenmeyers on ajoute 2 cm3 d'une solution, stérilisée par filtration sur membrane, de 10 mg de (pyridyl-3)-2 tétrahydrothiopyranne-(R,S) dans 2 cm3 d'eau contenant 4 % d'acide acétique.

La culture est poursuivie pendant 5 jours dans les mêmes conditions.

L'analyse par chromatographie en couche mince montre que le taux de transformation du (pyridyl-3)-2 tétrahydrothiopyranne est voisin de 60 %.

Dans chacun des 10 erlenmeyers, on ajoute 150 cm3 de méthanol et agite pendant 30 minutes. Après filtration et évaporation du méthanol sous pression réduite, la solution aqueuse résiduelle est percolée à travers une colonne contenant 20 g de silice greffée octadécyle (C₁₈). On lave avec de l'eau déminéralisée pour éliminer les sels minéraux puis élue les sulfoxydes avec 60 cm3 de méthanol. L'éluat méthanolique est concentré jusqu'à un volume de 5 cm3 puis est déposé sur une colonne (hauteur : 180 cm ; diamètre : 2,5 cm) contenant du Sephadex LH 20 (N.D. Pharmacia) montée dans le méthanol pur. On élue à un débit constant de 0,7 cm3/minute en recueillant des fractions de 5 cm3.

Les fractions 48 à 55 contiennent le (pyridyl-3)-2 tétrahydrothiopyranne-(R,S) non transformé, et les fractions 61 à 74, après évaporation du solvant, fournissent 80 mg d'un produit constitué, d'après l'analyse par chromatographie liquide à haute performance sur colonne chirale, d'un mélange des sulfoxydes (1R,2S) et (1R,2R).

Les sulfoxydes (1R,2S) et (1R,2R) peuvent être séparés par une nouvelle chromatographie sur colonne Séphadex LH 20 en effectuant l'élution à un débit de 0,25 cm3/minute et en recueillant des fractions de 5 cm3.

Les fractions 127 à 131 contiennent la forme (1R,2S) dont le pouvoir rotatoire, déterminé dans l'éthanol, est :
[α]²⁰_{D} = -198° ± 8°
Les fractions 138 à 142 contiennent la forme (1R,2R) dont le pouvoir rotatoire, déterminée dans l'éthanol, est :
[α]²⁰_{D} = -202° ± 5°
La présente invention concerne également les médicaments constitués par au moins un produit de formule générale (IV), à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits de l'invention sont particulièrement utiles dans le traitement de l'hypertension. Les doses dépendent de l'effet recherché et de la durée du traitement ; elles sont généralement comprises entre 5 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

### EXEMPLE

On prépare selon la technique habituelle des comprimés dosés à 25 mg de produit actif ayant la composition suivante :

| | |
|---|---|
| - N-méthyl (pyridyl-3)-2 tétrahydrothiopyraneecarbothioamide-2-oxyde-1-(1R,2R) | 25 mg |
| - amidon | 60 mg |
| - silice colloïdale | 50 mg |
| - stéréate de magnésium | 2 mg |

## Revendications (Revendications pour l'(les) Etat(s) contractant(s) suivant(s): AT, BE, CH, DE, DK, FR, GB, IT, LI, LU, NL, SE)

1. Procédé de préparation de (pyridyl-3)-2 tétrahydrothiopyrannecarbothioamide-2-oxydes-1-(1R,2R) de formule générale : 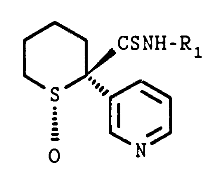 dans laquelle R₁ représente un radical alcoyle droit ou ramifié contenant 1 à 4 atomes de carbone, caractérisé en ce que l'on fait réagir un isothiocyanate d'alcoyle de formule générale :
S=C=N-R₁
dans laquelle R₁ est défini comme précédemment, sur des sulfoxydes 1R,2R et 1R,2S de formule : 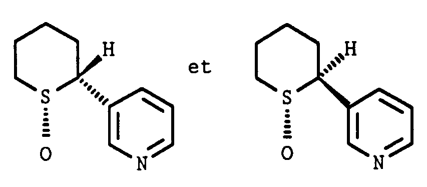 pris seuls ou en mélange, préalablement anionisés.

2. Procédé selon la revendication 1 caractérisé en ce que l'on fait réagir un isothiocyanate d'alcoyle en solution dans un solvant organique inerte sur les sulfoxydes pris seuls ou en mélange préalablement anionisés par action de l'amidure de sodium, éventuellement préparé in situ, dans l'ammoniac liquide à la température d'ébullition du mélange réactionnel c'est-à-dire voisine de -30°C.

3. Les sulfoxydes 1R,2R et 1R,2S de formule :

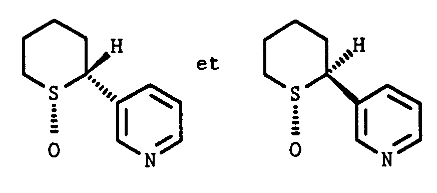

4. Un procédé de préparation des sulfoxydes selon la revendication 3 caractérisé en ce que l'on oxyde sélectivement un produit R,S de formule :

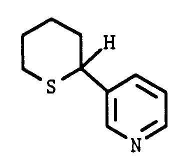

5. Procédé selon la revendication 4 caractérisé en ce que l'oxydation est réalisée par voie chimique au moyen d'un hydroperoxyde en présence d'un inducteur d'asymétrie tel que le (+) tartrate d'éthyle et d'un alcoolate de titane (IV) à une température voisine de -20°C.

6. Procédé selon la revendication 5 caractérisé en ce que l'hydroperoxyde est choisi parmi l'hydroperoxyde de cumyle et l'hydroperoxyde de tert.butyle.

7. Procédé selon la revendication 4 caractérisé en ce que l'oxydation est réalisée par voie biologique au moyen d'une culture d'un champignon filamenteux ou d'une bactérie filamenteuse ou au moyen d'une enzyme isolée en présence d'un agent d'oxydation.

8. Procédé selon la revendication 7 caractérisé en ce que l'oxydation est réalisée au moyen d'une culture d'Aspergillus foetidus NRRL 337.

## Revendications (Revendications pour l'(les) Etat(s) contractant(s) suivant(s): ES, GR)

1. Procédé de préparation de (pyridyl-3)-2 tétrahydrothiopyrannecarbothioamide-2-oxydes-1-(1R,2R) de formule générale : 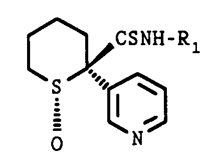 dans laquelle R₁ représente un radical alcoyle droit ou ramifié contenant 1 à 4 atomes de carbone, caractérisé en ce que l'on fait réagir un isothiocyanate d'alcoyle de formule générale :
S=C=N-R₁
dans laquelle R₁ est défini comme précédemment, sur des sulfoxydes 1R,2R et 1R,2S de formule : 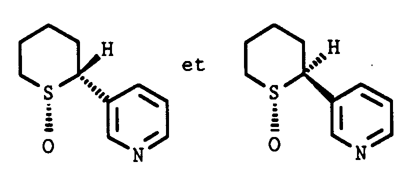 pris seuls ou en mélange, préalablement anionisés.

2. Procédé selon la revendication 1 caractérisé en ce que l'on fait réagir un isothiocyanate d'alcoyle en solution dans un solvant organique inerte sur les sulfoxydes pris seuls ou en mélange préalablement anionisés par action de l'amidure de sodium, éventuellement préparé in situ, dans l'ammoniac liquide à la température d'ébullition du mélange réactionnel c'est-à-dire voisine de -30°C.

3. Procédé de préparation des sulfoxydes 1R,2R et 1R,2S de formule : 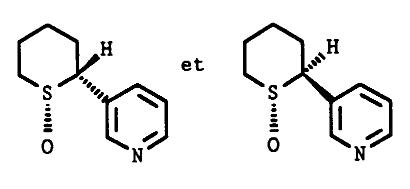 caractérisé en ce que l'on oxyde sélectivement un produit R,S de formule :

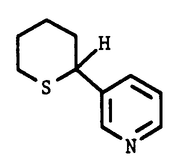

4. Procédé selon la revendication 3 caractérisé en ce que l'oxydation est réalisée par voie chimique au moyen d'un hydroperoxyde en présence d'un inducteur d'asymétrie tel que le (+) tartrate d'éthyle et d'un alcoolate de titane (IV) à une température voisine de -20°C.

5. Procédé selon la revendication 4 caractérisé en ce que l'hydroperoxyde est choisi parmi l'hydroperoxyde de cumyle et l'hydroperoxyde de tert.butyle.

6. Procédé selon la revendication 3 caractérisé en ce que l'oxydation est réalisée par voie biologique au moyen d'une culture d'un champignon filamenteux ou d'une bactérie filamenteuse ou au moyen d'une enzyme isolée en présence d'un agent d'oxydation.

7. Procédé selon la revendication 6 caractérisé en ce que l'oxydation est réalisée au moyen d'une culture d'Aspergillus foetidus NRRL 337.

## Claims (Claims for the following Contracting State(s): AT, BE, CH, DE, DK, FR, GB, IT, LI, LU, NL, SE)

1. Process for preparing (1R,2R)-2-(3-pyridyl)-tetrahydrothiopyran-2-carbothioamide 1-oxides of general formula: 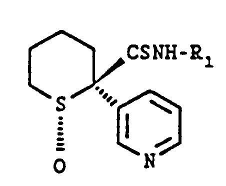 in which R₁ represents a linear or branched alkyl radical containing 1 to 4 carbon atoms, characterized in that an alkyl isothiocyanate of general formula:
S=C=N-R₁
in which R₁ is defined as above, is reacted with previously anionized 1R,2R and 1R,2S sulphoxides of formulae: 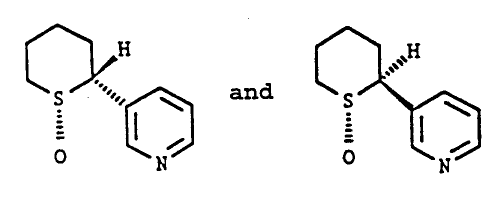 employed alone or mixed.

2. Process according to claim 1, characterized in that an alkyl isothiocyanate, dissolved in an inert organic solvent, is reacted with the sulphoxides employed alone or mixed, previously anionized by the action of sodium amide, optionally prepared in situ, in liquid ammonia at the boiling point of the reaction mixture, i.e. in the region of -30°C.

3. The 1R,2R and 1R,2S sulphoxides of formulae:

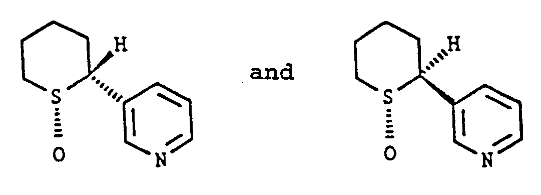

4. A process for preparing the sulphoxides according to claim 3, characterized in that an RS product of formula: 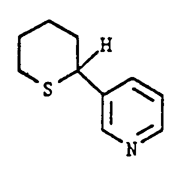 is oxidized selectively.

5. Process according to claim 4, characterized in that the oxidation is carried out chemically by means of a hydroperoxide in the presence of an asymmetry-inducing agent such as (+)-(ethyl tartrate) and a titanium(IV) alcoholate at a temperature in the region of -20°C.

6. Process according to claim 5, characterized in that the hydroperoxide is selected from cumyl hydroperoxide and tert-butyl hydroperoxide.

7. Process according to claim 4, characterized in that the oxidation is carried out biologically by means of a culture of a filamentous fungus or a filamentous bacterium, or by means of an enzyme isolated in the presence of an oxidizing agent.

8. Process according to claim 7, characterized in that the oxidation is carried out by means of a culture of Aspergillus foetidus NRRL 337.

## Claims (Claims for the following Contracting State(s): ES, GR)

1. Process for preparing (1R,2R)-2-(3-pyridyl)-tetrahydrothiopyran-2-carbothioamide 1-oxides of general formula: 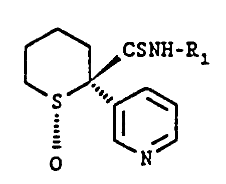 in which R₁ represents a linear or branched alkyl radical containing 1 to 4 carbon atoms, characterized in that an alkyl isothiocyanate of general formula:
S=C=N-R₁
in which R₁ is defined as above, is reacted with previously anionized 1R,2R and 1R,2S sulphoxides of formulae: 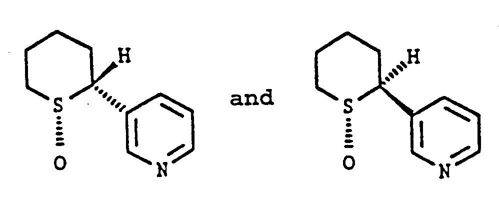 employed alone or mixed.

2. Process according to claim 1, characterized in that an alkyl isothiocyanate, dissolved in an inert organic solvent, is reacted with the sulphoxides employed alone or mixed, previously anionized by the action of sodium amide, optionally prepared in situ, in liquid ammonia at the boiling point of the reaction mixture, i.e. in the region of -30°C.

3. Process for preparing the 1R,2R and 1R,2S sulphoxides of formula : 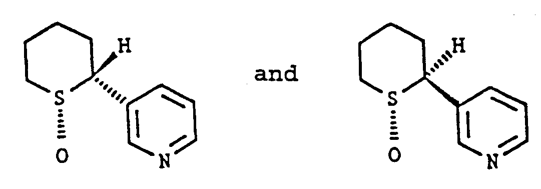 characterized in that an RS product of formula : 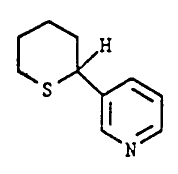 is oxidized selectively.

4. Process according to claim 3, characterized in that the oxidation is carried out chemically by means of a hydroperoxide in the presence of an asymmetry-inducing agent such as (+)-(ethyl tartrate) and a titanium(IV) alcoholate at a temperature in the region of -20°C.

5. Process according to claim 4, characterized in that the hydroperoxide is selected from cumyl hydroperoxide and tert-butyl hydroperoxide.

6. Process according to claim 3, characterized in that the oxidation is carried out biologically by means of a culture of a filamentous fungus or a filamentous bacterium, or by means of an enzyme isolated in the presence of an oxidizing agent.

7. Process according to claim 6, characterized in that the oxidation is carried out by means of a culture of Aspergillus foetidus NRRL 337.

## Patentansprüche (Patentansprüche für folgende(n) Vertragsstaat(en): AT, BE, CH, DE, DK, FR, GB, IT, LI, LU, NL, SE)

1. Verfahren zur Herstellung von 2-(3-Pyridyl)-tetrahydrothiopyran-2-carbothioamid-1-(1R,2R)-oxiden mit der allgemeinen Formel : 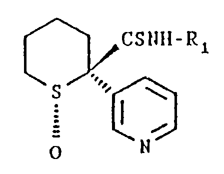 in der R₁ für einen geradlinigen oder verzweigten Alkylrest mit 1 bis 4 Kohlenstoffatomen steht, dadurch gekennzeichnet, daß man ein Alkylisoisothiocyanat mit der allgemeinen Formel:
S=C=N-R₁
in der R₁ wie vorher definiert ist, reagieren läßt mit 1R,2R- und 1R,2S-Sulfoxiden mit der Formel: 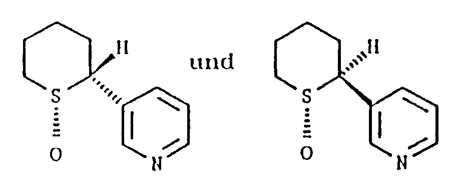 alleine eingesetzt oder in Mischung, die vorher in Anionen umgewandelt worden sind.

2. Verfahren gemäß Anspruch 1, dadurch gekennzeichnet, daß man ein Alkylisothiocycanat, im einem inerten organischen Lösungsmittel gelöst, mit den Sulfoxiden, die alleine oder in Mischung eingesetzt werden und vorher in Anionen umgewandelt worden sind, reagieren läßt durch Einwirkung von Natriumamid, das gegebenenfalls in situ hergestellt wird, in flüssigem Ammoniak bei der Siedetemperatur des Reaktionsgemischs, d.h. bei etwa -30°C.

3. Die 1R,2R- und 1R,2S-Sulfoxide mit der Formel:

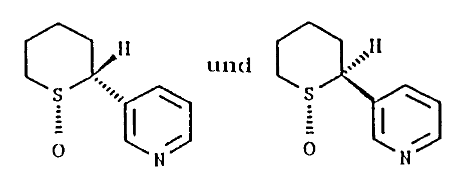

4. Ein Verfahren zur Herstellung der Sulfoxide gemäß Anspruch 3, dadurch gekennzeichnet, daß man ein R,S-Produkt mit folgender Formel selektiv oxidiert:

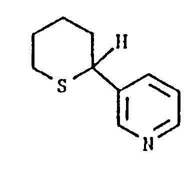

5. Verfahren gemäß Anspruch 4, dadurch gekennzeichnet, daß die Oxidation chemisch mit Hilfe eines Hydroperoxids durchgeführt wird in Gegenwart eines Asymmetrieinduktors wie dem Ethyl-(+)-tartrat und eines Titan(IV)-alkoholats bei einer Temperatur nahe -20°C.

6. Verfahren gemäß Anspruch 5, dadurch gekennzeichnet, daß das Hydroperoxid ausgewählt ist aus dem Cumylhydroperoxid und dem tert-Butylhydroperoxid.

7. Verfahren gemäß Anspruch 4, dadurch gekennzeichnet, daß die Oxidation biologisch mit Hilfe einer Kultur eines fadenförmigen Pilzes oder eines fadenförmigen Bakteriums oder mit Hilfe eines isolierten Enzyms in Gegenwart eins Oxidationsmittels durchgeführt wird.

8. Verfahren gemäß Anspruch 7, dadurch gekennzeichnet, daß die Oxidation mit einer Kultur von Aspergillus foetidus NRRL 337 durchgeführt wird.

## Patentansprüche (Patentansprüche für folgende(n) Vertragsstaat(en): ES, GR)

1. Verfahren zur Herstellung von 2-(3-Pyridyl)-tetrahydrothiopyran-2-carbothioamid-1-(1R,2R)-oxiden mit der allgemeinen Formel : 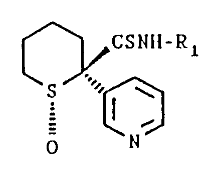 In der R₁ für einen geradlinigen oder verzweigten Alkylrest mit 1 bis 4 Kohlenstoffatomen steht, dadurch gekennzeichnet, daß man ein Alkylisoisothiocyanat mit der allgemeinen Formel:
S=C=N-R₁
in der R₁ wie vorher definiert ist, reagieren läßt mit 1R,2R- und 1R,2S-Sulfoxiden mit der Formel: 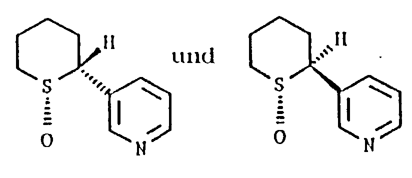 alleine eingesetzt oder in Mischung, die vorher in Anionen umgewandelt worden sind.

2. Verfahren gemäß Anspruch 1, dadurch gekennzeichnet, daß man ein Alkylisothiocycanal, in einem inerten organischen Lösungsmittel gelöst, mit den Sulfoxiden, die alleine oder in Mischung eingesetzt werden und vorher in Anionen umgewandelt worden sind, reagieren läßt durch Einwirkung von Natriumamid, das gegebenenfalls in situ hergestellt wird, in flüssigem Ammoniak bei der Siedetemperatur des Reaktionsgemischs, d.h. bei etwa -30°C.

3. Verfahren zur Herstellung der 1R,2R- und 1R,2S-Sulfoxide mit der Formel: 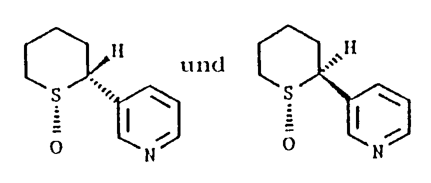 dadurch gekennzeichnet, daß man ein R,S-Produkt mit folgender Formel selektiv oxidiert:

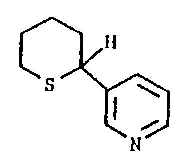

4. Verfahren gemäß Anspruch 3, dadurch gekennzeichnet, daß die Oxidation chemisch mit Hilfe eines Hydroperoxids durchgeführt wird in Gegenwart eines Asymmetrieinduktors wie dem Ethyl-(+)-tartrat und eines Titan(IV)-alkoholats bei einer Temperatur nahe -20°C.

5. Verfahren gemäß Anspruch 4, dadurch gekennzeichnet, daß das Hydroperoxid ausgewählt ist aus dem Cymylhydroperoxid und dem tert-Butylhydroperoxid.

6. Verfahren gemäß Anspruch 3, dadurch gekennzeichnet, daß die Oxidation biologisch mit Hilfe einer Kultur eines fadenförmigen Pilzes oder eines fadenförmigen Bakteriums oder mit Hilfe eines isolierten Enzyms in Gegenwart eines Oxidationsmittels durchgeführt wird.

7. Verfahren gemäß Anspruch 6, dadurch gekennzeichnet, daß die Oxidation mit einer Kultur von Aspergillus foetidus NRRL 337 durchgeführt wird.
